(19) Europäisches Patentamt
European Patent Office
Office européen des brevets

(11) EP 1 911 450 A1

(12) **EUROPEAN PATENT APPLICATION**
published in accordance with Art. 153(4) EPC

(43) Date of publication:
**16.04.2008 Bulletin 2008/16**

(21) Application number: **06761543.5**

(22) Date of filing: **21.07.2006**

(51) Int Cl.:
***A61K 31/565*** *(2006.01)* ***A61P 31/18*** *(2006.01)*

(86) International application number:
**PCT/CN2006/001808**

(87) International publication number:
**WO 2007/009397 (25.01.2007 Gazette 2007/04)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **22.07.2005 CN 200510028057**

(71) Applicant: **Shanghai Three-alliance Biotechnologies Co., Ltd.**
**Shanghai 200122 (CN)**

(72) Inventors:
- **WANG, Mingwei**
  **San Diego, CA 92129 (US)**
- **ZUO, Lin**
  **Shanghai 200122 (CN)**
- **YING, Minghua**
  **Zhejiang 317300 (CN)**

(74) Representative: **Comoglio, Elena et al**
**Jacobacci & Partners S.p.A**
**Corso Emilia, 8**
**10152 Torino (IT)**

# (54) USE OF CYMIPRISTONES FOR TREATING AIDS

(57) This invention refers to use of cymipristone type compounds of formula (I), salt or solvate thereof in preparation of medicament for treating AIDS. Cymipristone type compounds are a new type glucocorticoid receptor antagonist. Pharmacological test shows that cymipristone has anti-glucocorticoid activity and very strong affinity with glucocorticoid receptor. Test indicates that cymipristone is a glucocorticoid antagonist on receptor level and can block the function of glucocorticoid receptor. The inventor uses cymipristone to test the inhibition on the growth of AIDS virus in vitro and finds that cymipristone has significant growth inhibition action on AIDS pathogen HIV-1.

EP 1 911 450 A1

## Description

### Field of invention

This invention refers to pharmaceutical chemistry, in particular, use of cymipristone type compounds in AIDS treatment.

### Background technology

Acquired immunodeficiency syndrome (AIDS) is one of cosmopolitan important infectious diseases threatening human health and life. Human immunodeficiency virus (HIV), namely the pathogen of AIDS, is divided into two subgroups HIV-1 and HIV-2. HIV-1 is highly pathogenic and the main pathogen causing global epidemic of AIDS. And that HIV-2 has a lower transmission and pathogenicity, a longer latency and course of disease and a mild symptom, even, does not develop into AIDS in some infected patients. The most of AIDS patients are infected by HIV-1. HIV-2 infection is only localized in West Africa. So the current studies about AIDS virus mainly aim at HIV-1.

At the initial stage of HIV infection, viremia appears and there mild fever and lymphadenectasis. Afterwards, viremia reduces to a level difficult to be detected, antinuclear protein and antienvelope protein antibodies appear successively, and disease course enters into a symptomless carrier stage or latent infection period, which can be several years or even over 10 years, but up to now the mechanism of this long latency is not clear. Under the action of some factors, the virus can replicate in large quantities, and viremia appears again with AIDS symptoms, large quantity of helper T lymphocyte are destroyed by virus, immunological function of cell and body fluid decreases and becomes unable to resist invasion from outside pathogenic microorganisms, and finally the disease develops into AIDS.

The course of HIV proliferation can be divided generally into 9 steps, namely, adsorption, penetration, exuviation, initial stage protein synthesis, virus genome nucleic acid replication, late stage protein synthesis, nucleocapsid assembly, virus maturity, release, etc. Theoretically, each step in above course can be taken as the target for screening HIV medicaments. Through several years of efforts, corresponding inhibitors have been found for each phase involved in HIV proliferation and the different types of compounds have entered into preclinical or clinical study stage or have been approved by medicament administration department to market and to use in AIDS treatment. Now anti-AIDS medicaments that have been developed successfully include mainly: (1) reverse transcriptase inhibitor (as zidovudine, didanosine, zalcitabine, stavudine, lamivudine, abacavir, nevirapine, delavirdine, efavirenz, etc.) and (2) proteolytic enzyme inhibitor (as saquinavir, ritonavir, indinavir, nelfinavir, amprenavir, etc.). As HIV has high antigen variation characteristic and very easily produces medicament resistance, the single use of above medicaments will often fail in treatment on account of rapid production of medicament resistance. Although using reverse transcriptase inhibitor and proteolytic enzyme inhibitor simultaneously (cocktail therapy) can delay the appearance of medicament resistance and prolong the life of AIDS patient, the problem of medicament resistance cannot be radically solved and AIDS cannot be completely cured. Cocktail therapy may produce serious toxic side effect (as bone marrow inhibition, amnesia, etc.) and appearance of medicament resistant virus strain. Therefore, a new anti-HIV medicament of different chemical structure and action mechanism is urgently need clinically. Finding action target of new anti-HIV medicament is the key to realize this purpose..

The genome of HIV is mainly consists of genes Gag, Pol and Env. Besides, there are 6 supplementary genes (Tat, Vpr, Vpu, Nef, Rev and Vif), in which Vpr codes is a protein.Its molecular weight is about 14 kDa containing 96 amino acids.

Researches indicate that, after HIV infects host cell, Vpr interacts with several proteins in cell, and produces a series of influence on replication of virus, cycle and differentiation of host cell, for example, Vpr protein plays an important role in cell death caused by HIV As the pathogenic mechanism of HIV virus is in some respects abnormal regulation of cell induced by Vpr (Wei Qiang et al., "China Bioengineering Journal", 2003, vol. 23, 6), so Vpr protein has the potential of becoming a new target of anti-AIDS medicaments.

In studies on Vpr protein, scientists find that Vpr protein and glucocorticoid (as dexamethasone, hydrocortisone, etc.) can block cell proliferation in similar degree. So they conjecture that the inhibitory effect of Vpr on cell proliferation is at least partly similar to the action route of glucocorticoid (Ayyavoo et al., "Nature Medicine", 1997, vol. 3, 1117).

In the course of adjusting the immunological reaction of cell to different pathogens, several cellular factors mediate many important signal conduction function and play a key role in immunological reaction of cell. The glucocorticoid realizes its anti-inflammatory and immunological inhibition effect via interference on TCGF (interleukin 2) and synthesis or function of other cellular factors. Studies show that Vpr has also the action of interfering cellular factors. So it is conjectured that the anti-cell proliferation function of Vpr may depend partly on its inhibitory effect on cellular factors (including interleukin 2 and 12). As the initial reaction of cellular factors is very important for inhibiting the replication of HIV in infected cell, the negative effect of Vpr on cellular factors can destroy the immunological reaction of host, especially the immunological reaction of cell not yet infected by HIV-1 (Ayyavoo, V. et al., "Nature Medicine", 1997, vol. 3, 1117).

Experimental results indicate that glucocorticoid mediated by NF-kB reaction element inhibits the production

of cellular factors by inhibiting cellular factor promoter. Glucocorticoid induces the gene transcription of IkB, the translation formed IkB inhibits the activity of NF-kB by producing inactive NF-kB - IkB compound. Besides, all Vpr, either expressed by the cell itself or introduced by exogenous protein, can inhibit partly the gene activation mediated by NF-kB via inducting transcription of IkB. Since NF-kB is the key factor in activating cellular factor gene, blocking its function can effectively avoid or reduce the production of cellular factor, and this explains partly the action mechanism of Vpr.

Vpr protein, as analog of glucocorticoid, can adjust proliferation and death of cell and help replication of HIV in cell. Glucocorticoid receptor antagonist can reverse the action of Vpr, inhibit the replication of HIV in cell, and protect the cell to avoid entering into the cell death process mediated by Vpr (Ayyavoo, V. et al., "Nature Medicine" 1997, vol. 3, 1117). The inventor uses cymipristone, glucocorticoid receptor antagonist, to test the inhibition on growth of HIV virus in vitro and finds that cymipristone has significant growth inhibition action on HIV-1.

The Chinese patent (ZL 99 1 16829.1, Application No. CN 1287123A) granted for the inventor discloses the cymipristone type compounds of this invention, its preparation method and its use in preparation of medicament for treating diseases related to progestogen dependence, antifertility, abortion or contraception and antineoplasm,etc.. The patent is cited as reference in this application.

## Disclosure of the invention

The cymipristone type compounds involved in this invention are the steroid of following formula:

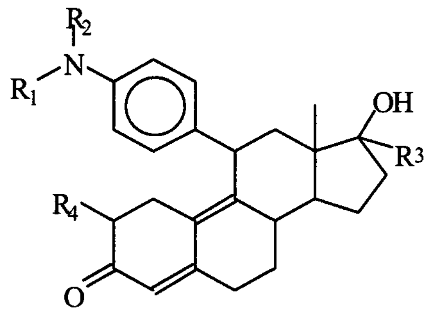

in which, $R_1$ is cyclopentyl, cyclohexyl or cycloheptyl; $R_2$ is H or $C_1$-$C_6$ alkyl; $R_3$ is selected from the group consisting of $-C\equiv C-R_5$ and $-CH=CH-R_6$, in which $R_5$ is H, $C_1$-$C_6$ alkyl or hydroxymethyl, $R_6$ is H, $C_1$-$C_6$ alkyl or hydroxymethyl; $R_4$ is H or hydroxymethylene (=CHOH).

The inventor has found that cymipristone type compounds are a new type glucocorticoid receptor antagonist. Pharmacological test shows that cymipristone has anti-glucocorticoid activity and cymipristone type compounds have very high affinity with glucocorticoid receptor. Above test proves that cymipristone is a glucocorticoid antagonist on receptor level and can block the function of glucocorticoid receptor.

The inventor uses cymipristone to test its inhibition on HIV growth in vitro. In MT-4 cell culture medium, the 50% inhibitory dose ($IC_{50}$) of cymipristone on HIV-1 IIIB P24 antigen is 1.30 $\mu$g/ml (2.62 $\mu$M). In PBMC cell culture medium, the 50% effective concentration ($EC_{50}$) of cymipristone on P24 antigen of HIV-1 AZT sensitive strain 018a is 12.36 $\mu$g/ml and that for P24 antigen of HIV-1 AZT resistant strain 018c is 7.90 $\mu$g/ml. This indicates that cymipristone has significant growth inhibition action on AIDS pathogen HIV-1. Thus, this invention is completed.

This invention provides the use of cymipristone type compounds, salt or solvate thereof in preparation of a medicament for treating AIDS.

The inventor has found that cymipristone type compounds can be used to treat AIDS. The cymipristone type compounds, salt or solvate thereof involved in this invention is a known steroid of following general formula (I):

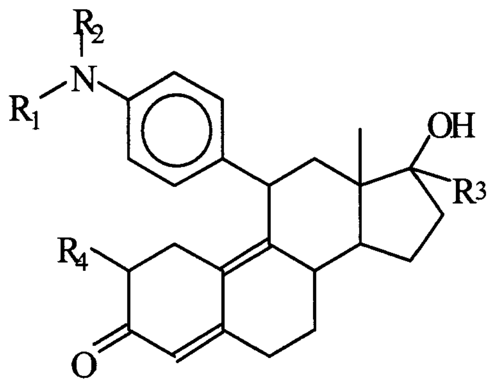

(I)

in which, $R_1$ is cyclopentyl, cyclohexyl or cycloheptyl; $R_2$ is H or $C_1$-$C_6$ alkyl; $R_3$ is selected from the group consisting of -C≡C-$R_5$ and -CH=CH-$R_6$, in which $R_5$ is H, $C_1$-$C_6$ alkyl or hydroxymethyl, $R_6$ is H, $C_1$-$C_6$ alkyl or hydroxymethyl; and $R_4$ is H or hydroxymethylene (=CHOH).

The compounds in this invention can be prepared according to the method disclosed in Chinese patent CN1287123A.

The compounds in this invention can exist in the form of salt or solvate. As it has several asymmetric carbon atoms, it has several isomers. All these salts and isomers belong to the use scope protected by this patent.

Among the compounds of formula (I) in this invention, the compound, in which $R_2$ is H or methyl, $R_3$ is -C≡C-$CH_3$ or -C≡C-$CH_2OH$, and $R_4$ is H, is preferable.

The more preferable compound is: 11β-[4-(N-methyl-N-cyclohexylamino)phenyl]-17α-(1-propynyl)-17β-hydroxyl-4,9-estradiene-3-one, namely cymipristone.

The inventor finds that cymipristone type compounds are a glucocorticoid receptor antagonist, has significant growth inhibition action on HIV- 1 and can be used to treat AIDS.

HIV has produced serious medicament-resistance against existing medicaments (including reverse transcriptase inhibitor and proteolytic enzyme inhibitor), but cymipristone type compounds have a mechanism of inhibiting HIV growth completely different from that of above medicaments. Therefore it can be used to treat not only diseases caused by non-resistant HIV infection, but also AIDS patients resistant to existing anti-HIV medicaments (including reverse transcriptase inhibitor and proteolytic enzyme inhibitor).

HIV is a complex reverse transcription virus and simultaneously using medicaments of different action mechanisms is an effective means for treating AIDS. In this invention, a more preferable implementation method is to use cymipristone type compound together with one or several of other medicaments that can treat HIV infection effectively, this medicament is preferable but not limited to reverse transcriptase inhibitor and/or proteolytic enzyme inhibitor. In which,the reverse transcriptase inhibitor is selected from zidovudine (AZT), didanosine (DDI), zalcitabine (DDC), stavudine (D4T), lamivudine (3TC), abacavir (ABC), nevirapine, delavirdine and efavirenz (EFV) and atc, one or several; the proteolytic enzyme inhibitor is selected from saquinavir, ritonavir, indinavir, nelfinavir , amprenavir and atc, one or several.

The therapeutic use of this invention can be implemented by the form of medicament combination. Therefore, this invention provides a medicament combination for treating AIDS, which can be prepared with conventional pharmaceutical technology. The medicament combination can be in the form of solid preparation as tablet, capsule, pill, granule, etc., and that of liquid preparation as injection, suspension, emulsion, solution, etc., or that of percutaneous preparation, including the preparation with special actions as sustained release, controlled release, target, pulse, etc.

**Embodiment**

The following examples further explain this invention. It should be understood that examples of this invention are used to explain and not to limit this invention and that all modifications based on essential of this invention are belong to the protection scope of this invention. Unless otherwise stated, all percentages in this invention are weight percentages.

Example 1: Preparation of cymipristone

Synthesis of cymipristone

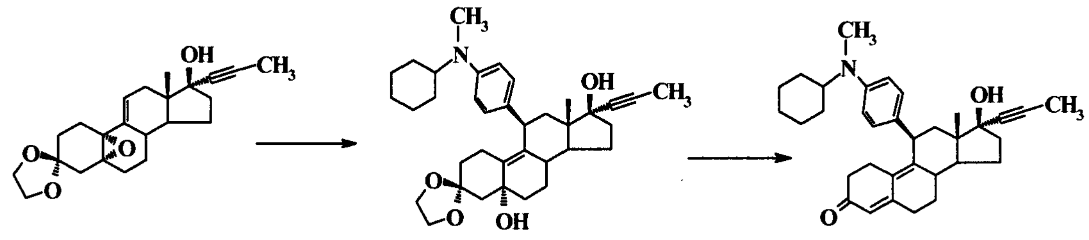

Epoxide Adduct cymipristone

(1) Preparation of 4-(N-methyl-N-cyclohexylamino)phenyl magnesium bromide

1.4g of magnesium tablet (Mg) and 10ml of anhydrous tetrahydrofuran (THF) are put into a four-neck flask, no adding iodine or adding a little quantity of iodine, 10.86g of 4-bromo-N-methyl-N-cyclohexylaniline (dissolved in 24ml of anhydrous tetrahydrofuran) are added dropwise into the flask at about 50°C. When the adding is completed, maintaining temperature and agitation are continued for 1 hour, and a solution of 4-(N-methyl-N-cyclohexylamino)phenyl magnesium bromide is obtained (for next step addition reaction) in tetrahydrofuran.

(2) Preparation of 3,3-ethylenedioxy-5α,17β-dihydroxy-11β-[4-(N-methyl-N-cyclohexylamino)phenyl]-17α-(1-propynyl)-9(10)-estrene (Adduct)

Put 5g of 3,3-ethylenedioxy-5,10-epoxy-17α-(1-propynyl)-17β-hydroxy-9(11)-estrene (epoxide), 29.1ml of anhydrous tetrahydrofuran (THF) and 0.1g of cuprous chloride ($Cu_2Cl_2$) into a four-neck flask, control temperature to below 5°C, the solution of 4-(N-methyl-N-cyclohexylamino)phenyl magnesium bromide in tetrahydrofuran is added dropwise into the flask. When the adding is completed, maintaining the temperature and reaction are continued for 5 hours. When the reaction is complete, the reaction liquid is poured into saturated aqueous solution of ammonium chloride, the water layer is separated. The organic layer is washed with saturated ammonium chloride solution and the water layer is extracted with ethyl acetate for several times. The organic layers are combined, and washed with saturated aqueous solution of sodium chloride, dried over anhydrous sodium sulfate, concentrated under reduced pressure, separated with silica gel column, eluting agent is cyclohexane : acetone =(5:1), and 6g of 3,3-ethylenedioxy-5α, 17β-dihydroxy-11β-[4-(N-methyl-N-cyclohexylamino)phenyl]-17α-(1-propynyl)-9(10)-estrene (Adduct) solid are obtained.
IR: (KBr) $cm^{-1}$: 3515 ($C_5$-OH, $C_{17}$-OH), 1612, 1515 (benzene ring), 819 (aromatic hydrogen).
$^1H$ NMR: ($CDCl_3$) δ ppm: 0.47 (3H, S, $C_{13}$-$CH_3$), 1.88 (3H, S, C ≡ C-$CH_3$), 2.72 (3H, S, N -$CH_3$), 6.65-7.03 (4H, ArH).

(3) Preparation of 11β-[4-(N-methyl-N-cyclohexylamino)phenyl]-17α-(1-propynyl) -17β-hydroxyl-4,9-estradiene-3-one (cymipristone)

2.5g of p-toluene sulfonic acid (PTS) and 5g of 3,3-ethylenedioxy-5α,17β-dihydroxy-11β-[4-(N-methyl-N-cyclohexylamino)phenyl]-17α-(1-propynyl)-9(10)-estrene (Adduct) are dissolved in 50ml of 90% ethyl alcohol (V/V) and reacted by agitation for 3 hours under 5-40°C. The reaction liquid is poured into diluted aqueous solution of sodium hydroxide, separate out solids, filter by suction and wash with water to neutral. The filter cake is dissolved in 50ml of ethyl acetate and washed with saturated aqueous solution of sodium chloride. The water layer is separated and a part of solvent is removed by distillation, separate out solid, filter by suction and dry, 3g of 11 β-[4-(N-methyl-N-cyclohexylamino)phenyl]-17α-(1-propynyl)-17β-hydroxyl-4,9-estradiene-3-one (cymipristone), light yellow solids, are obtained.
IR: (KBr) $cm^{-1}$: 3447 ($C_{17}$-OH), 1655 (unsaturated ketone), 1607, 1513 (benzene ring), 865, 819 (aromatic hydrogen).
$^1H$ NMR: ($CDCl_3$) δ ppm: 0.56 (3H, S, $C_{13}$-$CH_3$), 1.89 (3H, S, -C ≡ C-$CH_3$), 2.74 (3H, S, N -$CH_3$), 4.34 (1H, S, $C_{11}$-H), 5.75 (1H, S, $C_4$-H), 6.68-6.99 (4H, ArH).

Example 2: Determination of binding activity of cymipristone on human glucocorticoid receptor

Purpose: To determine the binding power of cymipristone on human glucocorticoid receptor and compare with mifepristone of similar chemical structure.

(1) Method

Buffer solution is composed of 25mM $NaPO_4$, 20mM $NaMoO_4$, 10mM KF and 10% glycerol (pH=7.3) and stored under 4°C. Stock solution is prepared with CHAPS and DTT and stored under -20°C. The solutions of positive drug (dexamethasone) and tested compound are prepared according to certain concentration gradient. Add 2.5 μ 1/hole into 96-pore plate. Take out appropriate amount of buffer solution reserve into solution to make the final concentration of CHAPS and DDT to become 0.25mM and 2mM respectively. Add protease inhibitors (aprotinin and leupeptin) to make its final concentration to become 1 μg/μl, and place in an ice bath. After adding human nuclear receptor GR (1:8), PR (1:100), ER (1:2000), AR (1:200) and MR (1:40) into the reaction system respectively, adequately mix suitable quantity of [$^3$H]-dexamethasone, [$^3$H]-progesterone, [$^3$H]-estradiol and [$^3$H]-DHT (from Amersham), rapidly add into 96-pore plate (100 μl/pore) and incubate overnight under 4°C. On the next day, add 25 μl of hydroxyapatite suspension into each pore and mix with shaking. After incubating for 10 min, separate by centrifuge for 3 min at 2500 rpm. The precipitate is washed with 100 μl of buffer solution and separated by centrifuge twice. Add 150 μl of scintillation solution (from Perkin Elmer) into each pore, mix with shaking and count by MicroBeta counter.

(2) Result

As shown in Table 1, the binding activity of cymipristone on glucocorticoid receptor (GR) is greatly higher than that of progestogen receptor (PR), estrogen receptor (ER), androgen receptor (AR) and mineralocorticoid receptor (MR), and the binding activity of cymipristone on human glucocorticoid receptor is more than that of mifepristone.

**Table 1. Binding activity of cymipristone on different nuclear receptors**

| (mean ± SD, n=4) | | |
|---|---|---|
| **Receptor** | **Cymipristone ($IC_{50}$, nM)** | **Mifepristone ($IC_{50}$, nM)** |
| GR | 0.45±0.069 | 0.63±0.056 |
| PRa | 5.2±0.26 | |
| ERα | 516.3±62.8 | |
| ERβ | 278.7±0.63 | |
| AR | 69.64±15.37 | |
| MR | >2000 | |

Example 3: Detection of binding activity of cymipristone on glucocorticoid receptor of rat hepatic cytoplasm

Purpose: To detect the binding power of cymipristone on animal glucocorticoid receptor and compare with mifepristone of similar chemical structure.

**(1) Method**

Clean female SD rats, body weight 200-250g, are fed with certain quantity of food and freely drink water. The temperature is controlled at about 24°C and an illumination cycle are for 11 hours by day and 13 hours at night. All animals are observed for 1 week before experiment.

Incisions are made on two sides below arch of ribs on dorsal position of rats to extirpate adrenal glands. After operation, the rats are fed with physiological saline to maintain water-electrolyte balance.

On the third day after operation, animals are acutely executed. The liver is taken out immediately and placed in ice cold buffer solution; the blood should be washed off as much as possible. Take certain quantity of tissue, add buffer solution (1:4), cut the tissue with scissors,intermittently homogenize the tissue under low temperature, centrifuge at 175000g for 1 hour, and take out the supernatant liquid as reserve.

Determine the concentration of protein in supernatant liquid and adjust the concentration of protein to 2mg/ml. evaporate to dry the labeled and non-labeled dexamethasone, cymipristone and mifepristone dissolved in ethyl alcohol

at 37°C. Then carry out the competition inhibition experiment.

Competition analysis: Take 0.2ml of cytoplasm, add [$^3$H]-dexamethasone of certain concentration as tracer. Add tested compounds (cymipristone, mifepristone and dexamethasone) of different concentrations, and incubate at 4°C for 24 hours. Use DCC to separate free and bound [$^3$H]-dexamethasone and determine the radioactivity of supernatant liquid and precipitate with liquid scintillation counter.

**(2) Data processing**

Relative binding affinity (RBA): calculate based on the standard of RBA of dexamethasone as 100%,

$$RBA=\frac{IC_{50}\text{ of dexamethasone}}{IC_{50}\text{ of corresponding compound}}$$

**(3) Result**

Competition inhibition experiment: each compounds can inhibit the binding of [$^3$H]-dexamethasone (7.87 nml/L) with glucocorticoid receptor of rat hepatic cytoplasm within the concentration range of 16 x 10-$^{10}$ ~ 5 x $10^{-7}$ mol/L. The inhibition rates are listed in Table 2.

**Table 2. Inhibition rate of cymipristone on binding of [$^3$H]-dexamethasone with glucocorticoid receptor of rat hepatic cytoplasm and comparison with mifepristone**

| (mean ±SD, %, n=4) | | | |
|---|---|---|---|
| Concentration of tested compounds (mol/L) | Dexamethasone | Cymipristone | Mifepristone |
| 5 x $10^{-7}$ | 89.23±8.56 | 95.36±5.34 | 93.84±3.78 |
| 1 x $10^{-7}$ | 60.37±5.67 | 88.94±9.01 | 88.95±5.69 |
| 2 x $10^{-8}$ | 39.42±4.83 | 75.60±3.56 | 70.24±4.13 |
| 4 x $10^{-9}$ | 24.83±5.92 | 54.54±4.24 | 46.84±7.25 |
| 8 x $10^{-10}$ | 14.72±7.34 | 31.25±3.87 | 24.43±3.97 |
| 16 x $10^{-10}$ | 10.37±6.11 | 17.25±6.76 | 16.74±8.07 |

Based on above calculation method, calculate the receptor pharmacological characteristic index of each compounds, see Table 3.

**Table 3. Relative binding activity (RBA) of cymipristone with glucocorticoid receptor of rat hepatic cytoplasm**

| (mean ±SD, n=4) | | |
|---|---|---|
| Compound | $IC_{50}$ (nmol/L) | RBA (%) |
| Dexamethasone | 14.50±1.89 | 100.00 |
| Cymipristone | 2.60±0.53 | 557.69±65.23 |
| Mifepristone | 4.21±1.02 | 344.41±57.41 |
| Note: Calculated based on the binding activity of dexamethasone as 100. | | |

RBA ratio of dexamethasone, cymipristone and mifepristone is 1:5.6:3.4. $IC_{50}$ are 14.50, 2.60 and 4.21 nmol/L respectively. $IC_{50}$ ratio of cymipristone and mifepristone is 1.00:1.62, indicating that cymipristone has very high binding power on glucocorticoid receptor and its binding power is stronger than that of mifepristone.The results from example 2 and 3 show that cymipristone is a stronger glucocorticoid receptor antagonist than mifepristone and has a greater

potential in treating AIDS.

Example 4: Antagonism of cymipristone on human glucocorticoid receptor

Purpose: To detect the antagonism of cymipristone on human glucocorticoid receptor.

**(1) Method**

Inoculate ($6x10^5$) of CV-1 cells into cell culture dish of 60mm diameter using phenol red-free low-sugar DMEM containing 10% FBS as culture medium and incubate overnight under 37 °C and 5% $CO_2$. Suction according to 1:5 ratio pipet 2 μg of plasmid carried GR genes and luciferase reporting genes, cotransfect CV-1 cells with FuGene 6 transfection reagent (from Roche), and incubate for 8 hous under above conditions. Inoculate the transfected cells into 96-pore micro culture plate, 8000 cells/100 μ l/pore, and incubate cells to adhibit on the wall of dish. Dilute the positive compound (Dexamethasone, final concentration 10 nM) and tested compound with culture medium according to certain concentration and add into above culture plate according to 10 μl/ pore, incubate for 24 hours. Discard the culture liquid, wash the cells twice with PBS, and pipet off all residual PBS. fragment the cells by CCLR Assay Kit (from Progema) for 15 min. Add 20 μl of fragmentation solution into 96 pore cell culture plate with white edge and white bottom, add CCLR substrate (from Progema), 100 μl/ pore, and immediately detect the chemical luminescence count value caused by activated luciferase on Wallac 1420 Multilabel Counter.

**(2) Result**

As shown in Table 4, cymipristone has a significant antagonistic activity on human glucocorticoid receptor function caused by dexamethasone in gene cotransfected live cells.

**Table 4. Antagonism of cymipristone on dexamethasone at cell level**

| (mean ±SD, n=4) | |
|---|---|
| Receptor | Cymipristone ($IC_{50}$, nM) |
| GR | 0.26±0.02 |

Example 5: Anti-glucocorticoid activity test for cymipristone

Purpose: To detect the anti-glucocorticoid activity of cymipristone.

**(1) Method**

Clean female SD rats, body weight 200-250g, are fed with certain quantity of food and freely drink water. The temperature is controlled at about 24 °C and an illumination cycle are for 11 hours by day and 13 hours at night. All animals are observed for 1 week before experiment.

Rats are anesthetized with ethyl ether. Incisions are made on two sides below costal arch of ribs on dorsal position of rats to extirpate adrenal glands. The rats are fed with physiological saline after operation. On the seventh day, animals are acutely executed. Take out thymus gland to place in to HBSS buffer solution, wash twice, remove connective tissue, cut the tissue with scissors, lightly grind with homogenizer, filter with three layers of gauze, and centrifuge the filtrate for 5 min (500 rpm). Wash the precipitated cells twice with HBSS, re-suspend the cells in RPMI1640 culture medium for cell count and cell survival rate determination, and adjust cell concentration to $1x10^7$/ml.

Determination of anti-glucocorticoid activity: Add tested compound into culture medium, reaction concentration $6x10^{-8}$~$2x10^{-6}$ mol/L, and place in 37 °C incubator for 3 hours. Add thymus gland cells to make cell concentration to become $10^7$/ml and incubate at 37 °C for 3 hours. Add 1 μ Ci of label substance, mix, and incubate for 1 hour. Add 100 μl of ice cold 5% trichloroacetic acid to end the reaction. Place the test tube on ice and wash twice with 2ml of ice cold 5% trichloroacetic acid and centrifuge twice, 500rpm x 5min. Add 0.5ml of 1N NaOH to dissolve the precipitated cells, pipet out the liquid, dry the residue with cotton stick, place into same scintillation bottle, and determine the radioactivity by liquid scintillation counter. In this test, dexamethasone of $6x10^{-8}$ mol/L concentration is selected to detect the anti-glucocorticoid activity, the concentration of tested compound is $6x10^{-8}$ ~ $2x10^{-6}$ mol/L.

**(2) Data processing**

Calculate the incorporation rate of [$^3$H]-TdR:

$$\text{Incorporation rate} = \frac{\text{Td - NSB}}{\text{T} - \text{NSB}} \text{ x } 100\%$$

In which: T=[$^3$H]-TdR count without presence of compound, Td=count with presence of compound, and NSB=non-specific adsorption.

The anti-glucocorticoid activity of cymipristone calculated acccording to the formula, includes the incorporation rate of [$^3$H]-TdR and residual inhibition rate, in which, residual inhibition rate is calculated as 100% when the concentration of dexamethasone is 6x10$^{-8}$ mol/L.

$$\text{Inhibition rate} = 100\% - \text{incorporation rate}$$

$$\textbf{Residual inhibition rate} = \frac{\textbf{Inhibition rate of tested compound combined with dexamethasone}}{\textbf{Inhibition rate of dexamethasone}}$$

**(3) Result**

When dexamethasone of 6 x 10$^{-8}$ mol/L concentration is used singly, incorporation rate of [$^3$H]-TdR is 43.2% ± 3.7%. When dexamethasone is combined with tested compound to use, its action receives different degrees of antagonism (Table 5). Residual inhibition rate shows the capability of cymipristone in the action of anti-glucocorticoid (Table 6).

**Table 5. Antagonism of cymipristone against dexamethasone**

| (Mean ± SD, %, n=4) | |
|---|---|
| Concentration (mol/L) | Cymipristone |
| 6x10$^{-8}$ | 80.0±4.3 |
| 2x10$^{-7}$ | 67.2±2.9 |
| 6x10$^{-7}$ | 76.0±3.8 |
| 2x10$^{-6}$ | 63.6±5.1 |

**Table 6. Effect of cymipristone on residual inhibition rate of dexamethasone**

| (Mean ± SD, %, n=4) | |
|---|---|
| Concentration (mol/L) | Cymipristone |
| 6x10$^{-8}$ | 35.2±2.4 |
| 2x10$^{-7}$ | 57.7±3.5 |
| 6x10$^{-7}$ | 42.2±1.9 |
| 2x10$^{-6}$ | 64.1±5.6 |

Result shows that cymipristone has anti-glucocorticoid activity.

Example 6: Growth inhibition action of cymipristone on HIV-1 in MT-4 cell culture

Purpose: To detect the anti-HIV-1 action of cymipristone.

**I. Method**

1. toxicity on cell and inhibition action on HIV-1 P24 antigen formation of cymipristone in MT-4 cell culture

(1) Inoculate MT-4 cells into 96-pore cell culture plate, $1 \times 10^5$ cells/ml, infect with HIV-1/IIIB virus strain of about $100 TCID_{50}$, and add cymipristone solution at the same time,its concentration is below 100μg/ml, triply diluted to 8 concentrations. Set virus $TCID_{50}$ determination group and cell control group. Each concentration is duplicated in two pore. Incubate under 37°C, 5% $CO_2$ and saturated humidity in an incubator.
(2) Determination of cytotoxicity of cymipristone by cytopathic method ($TC_{50}$ and $TC_0$)
Daily observe the cytopathy in above culture plate by microscope, record the degree of cytopathy with routine method, and calculate the 50% toxic concentration ($TC_{50}$) and maximum nontoxic concentration ($TC_0$) of drug according to Reed & Muench method.
(3) Determination on HIV-1 P24 antigen with supernatant liquid pipetted from cell culture plate added drug
After 4 days (96 hours), pipette out the supernatant liquid from cell culture added drug and freeze HIV- 1 P24 antigen used for determination.
(4) Determination of cytotoxicity of cymipristone by MTT staining method Add MTT into each pore of cell culture plate pipetted supernatant liquid to stain, continue to incubate for 4 hours, add decolorizing liquid, determine OD value at 570nm wavelength by enzyme-linked apparatus, and calculate $TC_{50}$ and $TC_0$ as above.
(5) Determination of HIV-1 P24 antigen and inhibitory action of cymipristone on HIV-1 P24 antigen
Take the supernatant liquid on the fourth day, combine the liquid from two pore, and dilute by 1:100. Determine the HIV- 1 P24 antigen titer (OD value) by ELISA method according to the instruction of HIV-1 P24 antigen reagent kit. Compare the OD value of tested drug group with that of virus control group, and calculate the inhibition %, 50% effective concentration ($IC_{50}$) and selection index(SI) of drug. At the same time, determine the 50% infection concentration of HIV-1/IIIB virus strain in MT-4 cells and virus infection quantity ($TCID_{50}$).

2. Calculation of 50% toxic concentration ($TC_{50}$), 50% effective concentration ($IC_{50}$) and selection index (SI)

(1) Calculation of $TC_{50}$, $IC_{50}$ and SI of drug in cell culture according to Reed & Muench method

$$TC_{50} \text{ or } IC_{50} = \text{antilog}\left(\log{<}50\%\text{ drug concentration} + \frac{50 - {<}50\%\text{ cumulative inhibition }\%}{{>}50\%\text{ cumulative inhibition }\% - {<}50\%\text{ cumulative inhibition }\%} \times \log\text{ dilution multiple}\right)$$

(2) $SI = TC_{50}/IC_{50}$

**II. Result**

1. Cytotoxicity of cymipristone in MT-4 cell culture liquid (Table 7)

(1) Cytotoxicity $TC_{50}$ from cytopathic method (CPE):
$TC_{50}$ of cymipristone is 7.704 μg/ml (15.50 μM).
(2) Cytotoxicity $TC_{50}$ from MTT staining method:
$TC_{50}$ of cymipristone is 30.26 μg/ml (60.89 μM).

**Table 7. Cytotoxicity of cymipristone in MT-4 cell culture**

| Determination method | Cymipristone ($TC_{50}$) |
|---|---|
| CPE | 7.704 μg/ml (15.50 μM) |
| MTT | 30.26 μg/ml (60.89 μM) |

**2. Inhibition action of cymipristone on HIV-1 IIIB P24 antigen in MT-4 cell culture (Table 8)**

In MT-4 cell culture, the inhibition activity ($IC_{50}$) of cymipristone on HIV-1 IIIB P24 antigen formation is 1.30 μg/ml (2.62 μM).

**Table 8. Inhibition action of cymipristone on HIV-1 P24 antigen in MT-4 cell culture**

| Concentration of cymipristone (μg/ml) | Inhibition rate (%) |
|---|---|
| 3.70 | 93 |
| 1.23 | 29 |
| 0.41 | 13 |
| 0.14 | 17 |
| 0.05 | 14 |
| $IC_{50}$ 1.30 μg/ml (2.62μM) | |

**3. Selection index (SI; Table 9)**

Calculate SI by CPE cytotoxicity: cymipristone is 5.93; calculate SI by MTT cytotoxicity: cymipristone is 23.28.

**Table 9. selection index (SI) of cymipristone inhibiting HFV-1 in MT-4 cell culture**

| Cytotoxicity determination method | Selection index (SI) |
|---|---|
| CPE | 5.93 |
| MTT | 23.28 |

**III. Conclusion**

1. Cymipristone has no significant toxicity on cultured human T-lymphocyte MT-4.
2. Cymipristone has significant inhibitory action on infection of HIV-1 virus IIIB experimental strain in passage MT-4 cell culture.
3. Cymipristone has significant inhibitory effect on HIV-1 P24 antigen formation in MT-4 cell culture.

Example 7: Inhibitory action of cymipristone on HIV-1 in PBMC cell culture

Purpose: To detect the inhibitory action of cymipristone on clinically separated AZT (zidovudine-sensitive HIV-1 018a and AZT-resistant HIV-1 018c virus strains in human peripheral blood mononuclear cell (PBMC) culture, and to compare with mifepristone of similar chemical structure.

I. Determination method of cytotoxicity and drug effect by adding drug after 2 hours of HIV virus infection

Take healthy human fresh venous blood, separate human peripheral blood mononuclear cells (PBMC) by FiColl separating liquid, prepare 1.5 x $10^5$ cells/ml suspension with culture solution containing PHA, inoculate in culture flask, and incubate under 37°C and 5% $CO_2$ for 72 hours in an incubator. Collect cells with curette, infect cells with $10^{-2}$ AZT sensitive (018a) or AZT resistant (018c) virus strain liquid, and adsorb for 2 hours. Wash off unadsorbed virus with 1640

culture medium without serum, prepare cells infected by virus to 1 x $10^6$/ml with culture solution, and inoculate on 96-pore culture plate, 100 μl/pore. At the same time,respectirely add 100 μl of 1:2 diluted cymipristone and mifepristone, and 1:5 diluted positive control drug AZT. Set cell control group and virus infected cell control group. Incubate under 37°C and 5% $CO_2$. On the fourth day (96 hours), pipette out the supernatant liquid and store under -20°C for determination of HIV-1 P24 antigen titer and calculation of drug inhibitory action. Add MTT staining to cells for determination of cytotoxicity.

Determination of cytotoxicity by MTT staining: Add 10 μl of 5mg/ml MTT stain into each pore, continue to incubate under 37°C, 5% $CO_2$ and saturated humidity for 4 hours. Add 100 μl of 50% DMF-17% Triton X-100 decolorizing solution into each pore,set overnight under 37°C, determine $OD_{570nm}$ value by enzyme-linked apparatus at 570nm wavelength, and calculate the 50% toxic concentration ($CC_{50}$) of drug. Determination of HIV-1 P24 antigen by ELISA method: thaw and dilute the supernatant liquid infected and drug-added PBMC cells and adjust dilution ratio according to pretest results. Determine the HIV- 1 P24 antigen titer according to the operation procedure provided by the HIV-1 P24 antigen reagent kit. Compare the drug-added group with virus control group. Calculate the 50% effective concentration ($EC_{50}$) of cymipristone, mifepristone or positive control drug AZT.

Calculation method of 50% toxic concentration ($CC_{50}$), 50% effective concentration ($EC_{50}$) and selection index (SI):

(1) Calculation method of inhibition rate of drug on cell or HIV-1 P24 antigen in cell culture

$$\text{Inhibition rate (\%)} = \frac{\text{Experimental group OD} - \text{Virus control group OD}}{\text{Normal control group OD} - \text{Virus control group OD}} \times 100$$

(2) Calculation method of $CC_{50}$ and $EC_{50}$ of drug in cell culture according to Reed & Muench method

$$CC_{50} \text{ or } EC_{50} = \text{antilog}\left(\log <50\% \text{ drug concentration} + \frac{50 - <50\% \text{ cumulative inhibition \%}}{>50\% \text{ cumulative inhibition \%} - <50\% \text{ cumulative inhibition \%}} \times \log \text{dilution multiple}\right)$$

(3) Calculation method of selection index (SI) on HIV-1 P24 antigen in cell culture SI = $CC_{50}/EC_{50}$

**II. Result**

1. Cytotoxicity and inhibition action on P24 antigen of cymipristone etc. in PBMC cell culture after infection by AZT-sensitive strain HIV- 018a for 2 hours.

(1) Cytotoxicity (Table 10)

**Table 10. Toxicity of cymipristone and mifepristone on PBMC cell culture infected by HIV-1 AZT-sensitive strain 018a**

| Drug | 50% toxic concentration on PBMC infected by AZT-sensitive strain 018a ($CC_{50}$, μg/ml) | | | |
|---|---|---|---|---|
| | Experiment 1 | Experiment 2 | Average | SD |
| Cymipristone | 103.62 | 156.05 | 129.84 | 37.07 |
| Mifepristone | 69.42 | 97.82 | 83.62 | 20.08 |
| AZT | 58.84 | 72.69 | 65.77 | 9.79 |

(2) Inhibition action on HIV-1 018a P24 antigen (Table 11)

**Table 11. Anti-HIV-1 018a action of cymipristone and mifepristone in PBMC** cells

| (administration after adsorbing for 2 hous) | | | | | | | | |
|---|---|---|---|---|---|---|---|---|
| Drug | $EC_{50}$ (μg/ml) | | | | SI | | | |
| | Experiment 1 | Experiment 2 | Average | SD | Experiment 1 | Experiment 2 | Average | SD |
| Cymipristone | 12.48 | 12.23 | 12.36 | 0.18 | 8.30 | 12.76 | 10.53 | 3.15 |
| Mifepristone | 12.40 | 12.37 | 12.38 | 0.02 | 5.60 | 7.91 | 6.76 | 1.63 |
| AZT | <0.0061 | <0.0064 | <0.0064 | 0 | 9193.75 | 11357.81 | 10275.78 | 1530.22 |

2. Cytotoxicity and inhibition action of on P24 antigen cymipristone etc. in PBMC cell culture after infection by AZT resistant strain HIV 018c for 2 hous.
(1) Cytotoxicity (Table 12)

**Table 12. Toxicity of cymipristone and mifepristone on PBMC cell culture infected by HIV-1 AZT-resistant strain 018c**

| Drug | 50% toxic concentration on PBMC infected by AZT-resistant strain 018c($CC_{50}$, μg/ml) | | | |
|---|---|---|---|---|
| | Experiment 1 | Experiment 2 | Average | SD |
| Cymipristone | 102.83 | 104.42 | 103.63 | 1.12 |
| Mifepristone | 105.02 | 74.69 | 89.86 | 21.45 |
| AZT | 70.72 | 77.90 | 74.31 | 5.08 |

(2) Inhibition action on AZT-resistant virus strain HIV- 018c P24 antigen (Table 13)

**Table 13. Anti-HIV-1 018c (AZT resistant strain) action of cymipristone and mifepristone in PBMC cells**

| (administration after adsorbing for 2 hous) | | | | | | | | |
|---|---|---|---|---|---|---|---|---|
| Drug | EC50 (μg/ml) | | | | SI | | | |
| | Experiment 1 | Experiment 2 | Average | SD | Experiment 1 2 | Experiment | Average | SD |
| Cymipristone | 8.42 | 7.38 | 7.90 | 0.74 | 12.21 | 14.15 | 13.18 | 1.37 |
| Mifepristone | 10.38 | 9.84 | 10.11 | 0.38 | 10.12 | 7.59 | 8.86 | 1.79 |
| AZT | 0.047 | 0.061 | 0.054 | 0.01 | 1504.68 | 1277.05 | 1390.87 | 160.96 |

**III. Conclusion:**

1. Cymipristone has inhibition action on P24 antigen caused by AZT sensitive and resistant HIV- 1 virus in PBMC cell culture.
2. Selection index of cymipristone inhibiting HIV-1 018a and HIV- 018c in PBMC cell culture is higher than that of mifepristone, suggesting that cymipristone has a better application prospect than mifepristone in treating AIDS.
3. Cymipristone has no cross resistance with currently clinically widely used zidovudine (AZT).

## Claims

**1.** Use of compound of formula (I) or salt or solvate thereof in preparation of a medicament for treating AIDS:

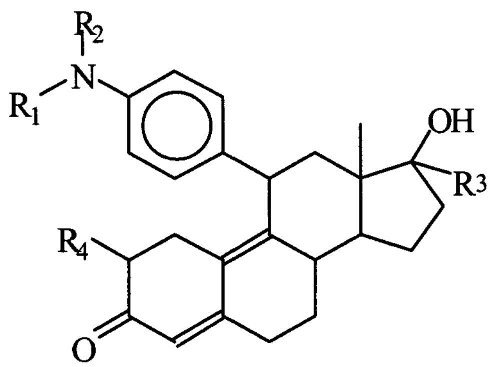

in which, $R_1$ is cyclopentyl, cyclohexyl or cycloheptyl; $R_2$ is H or $C_1$-$C_6$ alkyl; $R_3$ is selected from the group consisting of -C≡C-$R_5$ and -CH=CH-$R_6$, in which $R_5$ is H, $C_1$-$C_6$ alkyl or hydroxymethyl, $R_6$ is H, $C_1$-$C_6$ alkyl or hydroxymethyl; and $R_4$ is H or hydroxymethylene.

**2.** The use of claim 1, in which, in the compound of formula (I), $R_2$ is H or methyl, $R_3$ is -C≡C-$CH_3$ or -C≡C-$CH_2$OH, and $R_4$ is H.

**3.** The use of claim 2, in which the compound of formula (I) is 11 β-[4-(N-methyl-N-cyclohexylamino)phenyl]-17α-(1-propynyl)-17β-hydroxyl-4,9-estradiene-3-one.

**4.** The use of any one of claims 1-3, in which the compound of formula (I) or salt or solvate thereof is used in combination with reverse transcriptase inhibitor and/or proteolytic enzyme inhibitor.

**5.** The use of claim 4, in which, the reverse transcriptase inhibitor is one or more of the drugs selected from the group consisting of zidovudine (AZT), didanosine (DDI), zalcitabine (DDC), stavudine (D4T), lamivudine (3TC), abacavir (ABC), nevirapine, delavirdine, and efavirenz (EFV).

**6.** The use of claim 4, in which, the proteolytic enzyme inhibitor is one or more of the drugs selected from the group consisting of saquinavir, itonavir, indinavir, nelfinavir and amprenavir.

# INTERNATIONAL SEARCH REPORT

International application No.
PCT/CN2006/001808

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

IPC8: A61K, A61P, C07J, G01N, A01N

Documentation searched other than minimum documentation to the extent that such documents are included in the fields searched

Cprs, CTCMPD , CNKI Full-Text database, Chinese Medicine Abstract

Electronic data base consulted during the international search (name of data base and, where practicable, search terms used)

WPI, EPODOC, PAJ, Medline, CA, pristone, steroid, cortisol, hydrocortisone, glucocorticoid, phenyl, estradiene, AIDA, HIV

C. DOCUMENTS CONSIDERED TO BE RELEVANT

| Category* | Citation of document, with indication, where appropriate, of the relevant passages | Relevant to claim No. |
|---|---|---|
| X | WO 200411272 A2(VIRAL GENOMIX, INC) 29. Sep 2004, see descriptions, page 5, lines 11-20; page 8, lines 5-9; page 10, line 11-page 11, line 2; claims 1-2, 6-10 | 1-6 |
| X | US 5780220 A(TRUSTEES OF THE UNIVERSITY OF PENNSYLVANIA) 14. Jul 1998, see descriptions, column 6, lines 25-50; column 7, lines 10-16; column 12, lines 32-37; claim 3 | 1-6 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 21. Sep. 2006 | 26 · OCT 2006 (26 · 10 · 2006) |

| Name and mailing address of the ISA/CN | Authorized officer |
|---|---|
| The State Intellectual Property Office, the P.R.China<br>6 Xitucheng Rd., Jimen Bridge, Haidian District, Beijing, China 100088<br>Facsimile No. 86-10-62019451 | LEI, YaoLong<br>Telephone No. (86-10)62085254 |

Form PCT/ISA /210 (second sheet) (April 2005)

## INTERNATIONAL SEARCH REPORT

International application No.
PCT/CN2006/001808

CLASSIFICATION OF SUBJECT MATTER

A61K 31/565 (2006.01) i
A61P 31/18 (2006. 01) n

Form PCT/ISA /210 (extra sheet) (April 2005)

**INTERNATIONAL SEARCH REPORT**
Information on patent family members

International application No.
PCT/CN2006/001808

| Patent Documents referred in the Report | Publication Date | Patent Family | Publication Date |
|---|---|---|---|
| WO 2004112724 A2 | 29. 12. 2004 | CA2529852 A | 29. 12. 2004 |
| | | AU2004249295 A | 29. 12. 2004 |
| | | WO2004112724 A | 29. 12. 2004 |
| | | EP1643946 A | 12. 04. 2006 |
| | | EP20040755774 | 21. 06. 2004 |
| US 5780220 A | 14. 07. 1998 | WO9531901 A | 30. 11. 1995 |
| | | CA2190613 A | 30. 11. 1995 |
| | | AU2588095 A | 18. 12. 1995 |
| | | EP0759693 AB | 05. 03. 1997 |
| | | EP19950920426 | 17. 05. 1995 |
| | | US5639598 A | 17. 06. 1997 |
| | | AU690694B | 30. 04. 1998 |
| | | US5780220A | 14. 07. 1998 |
| | | AT254847T | 15. 12. 2003 |
| | | DE69532197D | 08. 01. 2004 |
| | | PT759693T | 31. 03. 2004 |
| | | DE69532197T | 15. 04. 2004 |
| | | ES2210291T | 01. 07. 2004 |

Form PCT/ISA /210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN ZL99116829
- CN 1287123 A


### Non-patent literature cited in the description


- **WEI QIANG et al.** *China Bioengineering Journal,* 2003, vol. 23, 6
- **AYYAVOO et al.** *Nature Medicine,* 1997, vol. 3, 1117
- **AYYAVOO, V. et al.** *Nature Medicine,* 1997, vol. 3, 1117